Europäisches Patentamt

(19) European Patent Office

Office européen des brevets

(11) Veröffentlichungsnummer: **0 158 277**
**A2**

(12) ## EUROPÄISCHE PATENTANMELDUNG

(21) Anmeldenummer: 85104057.6

(22) Anmeldetag: 03.04.85

(51) Int. Cl.⁴: **A 61 K 9/22**
**A 61 K 37/24**

(30) Priorität: 11.04.84 DE 3413608

(43) Veröffentlichungstag der Anmeldung:
16.10.85 Patentblatt 85/42

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Sandow, Jürgen Kurt, Dr.
Am Haideplacken 22
D-6240 Königstein/Taunus(DE)

(72) Erfinder: Seidel, Heinz-Rüdiger, Dr.
Im Kirschenfeld 15
D-6370 Oberursel(DE)

(54) Implantierbare Zubereitungen von regulatorischen Peptiden mit gesteuerter Freisetzung und Verfahren zur ihrer Herstellung.

(57) Implanierbare Zubereitungen von regulatorischen Peptiden mit gesteuerter Wirkstoff-Freigabe, enthaltend als Trägerstoff Zein sowie Verfahren zur Herstellung solcher Implantante werden beschrieben.

EP 0 158 277 A2

HOECHST AKTIENGESELLSCHAFT     HOE 84/F 083     Dr.D/cr

Implantierbare Zubereitungen von regulatorischen Peptiden mit gesteuerter Freisetzung und Verfahren zu ihrer Herstellung

Die Erfindung betrifft implantierbare Zubereitungen von regulatorischen Peptiden mit gesteuerter Wirkstoff-Freigabe, welche als biologisch abbaubaren Trägerstoff Zein enthalten, sowie Verfahren zur Herstellung solcher Implantate.

Implantate dienen der lang anhaltenden gesteuerten Freisetzung von Wirkstoffen zur Erzielung konstanter Blutspiegel. Der Wirkstoff ist dabei in eine Matrix aus natürlichen oder synthetischen Polymeren eingebettet. Er kann gelöst oder fein verteilt darin vorliegen. Seine Freisetzung erfolgt durch Diffusion, durch Erosion oder durch beide Prozesse gleichzeitig. Die Erosion spielt dann eine Rolle, wenn der Trägerstoff biologisch abbaubar ist. Bekannte zur Implantation geeignete polymere Trägerstoffe sind z.B. Siliconpolymere und Ethylen-Vinylacetat-Copolymere. Während Implantate aus diesen Kunststoffen nach Freisetzung des Wirkstoffs chirurgisch wieder entfernt werden müssen, entfällt diese Maßnahme bei biologisch abbaubaren Polymeren. Derartige biodegradable Polymere stellen z.B. Polymilchsäure und Copolymere aus Milchsäure und Glykolsäure oder Polyalkyl-2-cyanacrylate dar. Biologisch abbaubar ist ferner die aus Bakterien gewonnene Poly-3-hydroxybuttersäure. Natürlichen Ursprungs und ebenfalls biodegradabel sind Gelatine und Albumin, die durch Hitzebehandlung oder chemische Vernetzung gehärtet werden müssen.

Die genannten Implantatmaterialien, die für eine parenterale Verabreichung verwendbar sein sollen, sind nicht als ideal zu bezeichnen:

Sie können unverträgliche Nebenprodukte beim Abbau im Körper bilden; so kann z.B. im Falle der Polybutyl-2-cyanacrylate beim Abbau Butanol entstehen. Bei synthetischen Polymeren ist mit Beimengungen von Polymerisationskatalysator-Rückständen zu rechnen. Aus Implantaten auf Basis von Gelatine wird der Wirkstoff meist zu schnell abgegeben. Zu lange Freisetzungszeiten ergeben sich u.U. bei Anwendung von Poly-3-hydroxybuttersäure. Bei nicht biologisch abbaubaren Materialien ist eine nachträgliche chirurgische Entfernung des Implantats erforderlich.

Es wurde nun gefunden, daß sich Zein als Trägerstoff für Implantate enthaltend Peptide als Wirkstoff eignet.

Zein ist ein pflanzlicher Eiweißstoff und wird aus Mais gewonnen. Von Vorteil ist, daß seine biologische Abbaubarkeit im Bereich von einigen Wochen liegt. Es ist ein Naturprodukt, das gut zugänglich, leicht erhältlich und relativ preiswert ist. Zein gehört zu den Prolaminen, welche physiologisch unbedenkliche Substanzen sind. Alkoholische Zein-Lösungen zur parenteralen Applikation wurden bereits beschrieben (deutsche Patentschrift 28 03 869). Diese Lösungen enthalten zusätzlich ein Öl. Die Herstellung ist aufwendig, die Stabilität der erhaltenen Emulsionen ist nicht unbedingt gewährleistet.

Die Erfindung betrifft daher Implantate enthaltend regulatorische Peptide als Wirkstoffe und Zein als biologisch abbaubaren Trägerstoff.

In den vorstehenden und nachfolgenden Ausführungen steht "Peptide" für regulatorische Peptide, deren Analoga sowie deren physiologisch verträgliche Salze. Sie sind natürlichen, synthetischen oder halbsynthetischen Ursprungs.

Unter Implantaten oder implantierbaren Zubereitungen werden feste Depotarzneiformen verstanden, wobei jeder einzelne Formkörper eine einzelne, für sich implantierbare Arzneiform, wie z.B. eine Tablette, darstellt, im Gegensatz zur Sammelarzneiform (z.B. Vereinigung von vielen Mikrokapseln für Insektionszwecke).

Regulatorische Peptide, auch als Peptidhormone bezeichnet, sind physiologisch wirksme endogene Peptide. Sie sind z.B. in Wasser und niedermolekularen Alkoholen gut löslich. Als Vertreter, die die erfindungsgemäßen Implantate enthalten können, seien genannt Oxytocin, Vasopressin, Adrenocorticortropes Hormon, Prolactin, Luteinisierendes Hormon-Releasing Hormon (LH-RH), Insulin, Glucagon, Gastrin, Sekretin und Somatostatin.

Von besonderer Bedeutung sind die LH-RH-Analoga, wie z.B. das Buserelinacetat, die bei kontinuierlicher Verabreichung durch Blockade der Hypophyse zu einer stark verringerten Ausschüttung der gonadotropen Hormone führen und damit zu einer Hemmung der Sekretion von Östradiol und Testosteron. Diese Wirkungen lassen sich zur Behandlung des Mammacarcinoms, der Endometriose, des Prostatacarcinoms und der Pubertas praecox nutzen.

Die zur Implantation bestimmte Zubereitung besteht aus einer Zein-Matrix, in der die Peptide eingebettet sind. Ihre Freisetzung erfolgt durch Diffusion und Erosion. Eine Steuerung der Freisetzung ist einmal durch den Anteil an Peptid in dem Implantat möglich. Das Verhältnis Wirkstoff zu Zein beträgt vorzugsweise 1 : 10 bis 1 : 1000, insbesondere 1 : 10 bis 1 : 100.

Durch Zusatz hydrophiler Stoffe läßt sich die Freigabe beschleunigen. Umgekehrt läßt sich die Freigabe verlängern, indem die Implantate mit einem die Freisetzung verzögerndem Überzug versehen werden.

Die peptidhaltigen Zein-Implantate können also noch weitere Stoffe enthalten. Als hydrophile Zusätze eignen sich z.B. Zucker wie Milchzucker, Polyalkohole wie z.B. Mannit, Aminosäuren wie Glykokoll und Proteine wie beispielsweise Gelatine.

Als Filmbildner für eine Retardhülle kommen vor allem das Zein selbst als biologisch abbaubarer Stoff in Frage, weiterhin andere biodegradable Polymere wie Polymilchsäure, Copolymere der Milch- und Glykolsäure und Poly-3-hydroxybuttersäure. Daneben lassen sich auch nicht abbaubare Polymere wie Ethylcellulose oder Poly(meth-)acrylsäurederivat verwenden, wobei dann allerdings eine Reoperation des Implantats erforderlich ist. Als Filmbildner ist Zein bevorzugt.

Die Erfindung betrifft weiterhin Verfahren zur Herstellung von implantierbaren Zubereitungen aus Zein zur gesteuerten Freisetzung von Peptiden.

Das Verfahren zur Herstellung der Implantate ist dadurch gekennzeichnet, daß man Peptide und Zein innig vermischt und, gegebenenfalls nach Granulation, zu Formkörpern verpreßt.

Die Preßlinge können ganz unterschiedliche Formen besitzen wie Tabletten, Dragees, Oblongtabletten oder Pellets. Vor dem Verpressen können hydrophile Stoffe zugemischt werden. Die Preßlinge können mit einem Filmbildner überzogen werden.

Eine bevorzugte Ausführungsform des Verfahrens besteht darin, Zein und Peptid in einem Gemisch aus Wasser und einem $(C_2-C_4)$-Alkohol, vorzugsweise einem Äthanol-Wasser-Gemisch zu lösen, in diese Lösung gegebenenfalls hydrophile Zusätze zu lösen oder zu suspendieren, und die Lösung bzw. Suspension zu sprühtrocknen. Das sprühgetrocknete Material wird verpreßt. Wenn gewünscht, werden die Preßlinge durch Filmlackierung mit einem Überzug versehen. Die hydrophilen Stoffe können auch nach der Sprühtrocknung zugesetzt werden.

Die geschilderten Maßnahmen erlauben Implantate mit verschiedener Lebensdauer, d.h. mit unterschiedlichen Freisetzungszeiträumen entsprechend der therapeutischen Notwendigkeit herzustellen.

Für die Behandlung der Patienten ist es wichtig, daß die Peptide aus der implantierbaren Zubereitung gleichmäßig über mehrere Wochen freigegeben werden. Mit der erfindungsgemäßen Zubereitung mit Zein gelingt es, die Wirkstoff-Freigabe in gewünschter Weise zu kontrollieren.

Die Freigabe des Peptids aus einem erfindungsgemäßen Implantat erfolgt innerhalb eines überschaubaren Zeitraums, so daß auch eine Kontrolle der Patienten durch den Arzt leichter möglich ist. Gewünschtenfalls kann sich an eine Behandlung mit einem erfindungsgemäßen, relativ kurz wirksamen Implantat die Behandlung mit einem langwirksamen anschließen.

Die folgenden Beispiele sollen die Erfindung näher erläutern:

Beispiel 1

10 g Zein und 11,5 mg Buserelinacetat (Gehalt an Base 87 %) wurden in einer Mischung aus 140 ml Ethanol und 60 ml Wasser gelöst und sprühgetrocknet. Man erhielt ein gelbliches Pulver, das zu 50 mg schweren Tabletten mit einem Gehalt an 50 µg Buserelin verpreßt wurde.

Beispiel 2

4,5 g Zein und 5,75 mg Buserelinacetat (Gehalt an Base
87 %) wurden in einer Mischung aus 70 ml Ethanol und
30 ml Wasser gelöst und sprühgetrocknet. Man erhielt ein
gelbliches Pulver, das mit 500 mg Milchzucker gemischt
wurde. Die Mischung wird zu 50 mg schweren Tabletten mit
einem Gehalt von 50 µg Buserelin verpreßt.

Beispiel 3

1,431 g Zein und 69 mg Buserelinacetat (Gehalt an Base
87 %) wurden in einer Mischung aus 70 ml Ethanol und
30 ml Wasser gelöst und sprühgetrocknet. Man erhielt ein
gelbliches Pulver, das zu 50 mg schweren drageeförmigen
Kernen mit einem Gehalt von 2 µg Buserelin verpreßt
wurde. Aus technischen Gründen wurden diese Kerne gemeinsam mit 900 g Placebokernen z.B. aus Milchzucker durch
Besprühen mit 750 g einer Lösung von 100 g Zein in 900 g
70% Ethanol filmlackiert. Anschließend wurden die Buselin enthaltenden Filmtabletten ausgelesen. Die Überzugsmenge betrug ca. 3 mg pro filmlackiertem Zein-Wirkstoff-
Kern.

Beispiel 4

Daß die erfindungsgemäßen Zubereitungen eine lang anhaltende gleichmäßige Freisetzung bewirken, wurde an den
gemäß Beispiel 1 hergestellten Buserelin-Zein-Implantaten
sowohl in vitro als auch in vivo beobachtet.

In vitro-Freisetzung (1 ml physiologische Kochsalzlösung,
Raumtemperatur, täglicher Mediumwechsel): Nach einem
hohen Freisetzungsstoß zu Beginn (innerhalb der ersten
2-3 Tage) verlief die Freisetzung bis zum 27. Tag gemessen, relativ konstant. Die mittlere Freisetzungsrate
betrug duchschnittlich 243 ng/Tag.

In vivo-Freisetzung (nach Implantation in das Subcutangewebe bei Ratten, Messung der Buserelin-Exkretion in den
Urin durch RIA): Es stellte sich ein ziemlich gleichbleibender Spiegel von 100 ng-300 ng/Tag ein. Unter der Annahme, daß 30 % der täglich freigesetzten Buserelinmenge
in dem Urin ausgeschieden wurden, wie dies bei Dauerinfusionen zu beobachten ist, ergab sich eine in vivo-
Freisetzungsrate von durchschnittlich 567 ng/Tag.

PATENTANSPRÜCHE:

1. Implantierbare Zubereitung mit gesteuerter Wirkstoff-Freigabe, dadurch gekennzeichnet, daß sie als Wirkstoff ein regulatorisches Peptid, eines seiner Analoga oder eines seiner physiologisch verträglichen Salze als Wirkstoff und Zein als Trägerstoff enthält.

2. Implantierbare Zubereitung gemäß Anspruch 1, dadurch gekennzeichnet, daß sie einen hydrophilen, pharmakologisch verträglichen Zusatzstoff enthält.

3. Implantat gemäß Anspruch 1, dadurch gekennzeichnet, daß es einen die Wirkstoff-Freigabe verzögernden Film aufweist.

4. Implantat gemäß Anspruch 1, dadurch gekennzeichnet, daß es einen die Freigabe verzögernden Zein-Filmüberzug aufweist.

5. Implantat gemäß Anspruch 1, dadurch gekennzeichnet, daß es Buserelin oder ein physiologisch verträgliches Buserelin-Salz als Wirkstoff enthält.

6. Verfahren zur Herstellung einer implantierbaren Zubereitung gemäß Anspruch 1, dadurch gekennzeichnet, daß man den Wirkstoff und Zein innig vermischt und die Mischung, gegebenenfalls nach vorheriger Granulation, zu Formkörpern verpreßt.

7. Verfahren gemäß Anspruch 6, dadurch gekennzeichnet, daß man Zein und Wirkstoff in einem Gemisch aus Wasser und einem $(C_2-C_4)$-Alkohol, vorzugsweise aus Wasser und Ethanol löst und sprühtrocknet und zu Formlingen verpreßt.

8. Verfahren gemäß Anspruch 7, dadurch gekennzeichnet,
   daß man entweder der Lösung oder dem sprühgetrockneten
   Material hydrophile Stoffe zusetzt.

9. Verfahren gemäß Anspruch 7, dadurch gekennzeichnet,
   daß man die Formkörper mit einem Zein-Film überzieht.

Patentansprüche für Österreich                    HOE 84/F 083

1. Verfahren zur Herstellung einer implantierbaren Zubereitung mit gesteuerter Wirkstoff-Freigabe, dadurch gekennzeichnet, daß man ein regulatorisches Peptid, eines seiner Analoga oder eines seiner physiologisch verträglichen Salze (Wirkstoff) und Zein (Trägerstoff) innig vermischt und die Mischung, gegebenenfalls nach vorheriger Granulation, zu Formkörpern verpreßt.

2. Verfahren gemäß Anspruch 1, dadurch gekennzeichnet, daß man Zein und Wirkstoff in einem Gemisch aus Wasser und einem $(C_2-C_4)$-Alkohol, vorzugsweise aus Wasser und Ethanol, löst und sprühtrocknet und zu Formlingen verpreßt.

3. Verfahren gemäß Anspruch 2, dadurch gekennzeichnet, daß man entweder der Lösung oder dem sprühgetrockneten Material hydrophile, pharmakologisch verträgliche Stoffe zusetzt.

4. Verfahren gemäß Anspruch 2, dadurch gekennzeichnet, daß man die Formkörper mit einem die Freigabe verzögernden Film, vorzugsweise einem Zein-Film, überzieht.

5. Verfahren gemäß Anspruch 1 oder 2, dadurch gekennzeichnet, daß man als Wirkstoff Buserelin oder ein physiologische verträgliches Buserelin-Salz einsetzt.